# EUROPÄISCHE PATENTSCHRIFT

(11) **EP 3 629 917 B1**
(45) Veröffentlichungstag und Bekanntmachung des Hinweises auf die Patenterteilung: **21.07.2021**
(21) Anmeldenummer: 18728562.2
(22) Anmeldetag: 24.05.2018
(51) Int. Cl.: A61B 5/08, G01N 27/62

(54) **ANALYSEVORRICHTUNG ZUM ANALYSIEREN VON EXSPIRATIONSLUFT**
ANALYSIS DEVICE FOR ANALYZING EXPIRATION AIR
DISPOSITIF D'ANALYSE POUR ANALYSER L'AIR EXPIRÉ

(30) Priorität: 24.05.2017 DE 102017111459
(43) Veröffentlichungstag der Anmeldung: 08.04.2020
(73) Patentinhaber: B. Braun Melsungen AG, 34212 Melsungen (DE)
(72) Erfinder: SPRAVE-BAUMBACH, Gabriele, 72762 Reutlingen (DE)
(74) Vertreter: Winter, Brandl - Partnerschaft mbB
(86) Internationale Anmeldenummer: PCT/EP2018/063720
(87) Internationale Veröffentlichungsnummer: WO 2018/215618

(56) Entgegenhaltungen:
- DE-A1-102013 112 921
- DE-U1-202013 105 685
- US-A1- 2009 166 524
- WOLFGANG VAUTZ ET AL: "Breath analysis-performance and potential of ion mobility spectrometry", JOURNAL OF BREATH RESEARCH, Bd. 3, Nr. 3, 1. September 2009 (2009-09-01), Seite 036004, XP055508835, US ISSN: 1752-7155, DOI: 10.1088/1752-7155/3/3/036004

## Beschreibung

Die Erfindung betrifft eine Analysevorrichtung zum Analysieren von Exspirationsluft eines Patienten, vorzugsweise zum Überwachen einer Narkose des Patienten während eines medizinischen Eingriffs, wobei die Analysevorrichtung konfiguriert ist, einen Anteil eines in der Exspirationsluft des Patienten enthaltenen Analyten in der Exspirationsluft zu bestimmen, mit: vorzugsweise einer Multikapillarsäule zum Vortrennen der zu analysierenden Exspirationsluft; und einem lonenbeweglichkeitsspektrometer, in welchem Gasbestandteile der Exspirationsluft ionisiert und hin zu einer Detektionsvorrichtung beschleunigt werden; wobei die Analysevorrichtung Signalausschläge ausgibt, welche durch die auf die Detektionsvorrichtung treffenden, ionisierten Gasbestandteile der Exspirationsluft erzeugt werden.

### Stand der Technik

Es sind aus dem Stand der Technik lonenbeweglichkeitsspektrometer bekannt, welche einer Detektion von chemischen Substanzen, Kampfstoffen, Sprengstoffen, Drogen, etc. dienen. Auch ist es beispielsweise aus der DE 20 2013 105 685 U1 bekannt, solche lonenbeweglichkeitsspektrometer in der Medizin, beispielsweise in der Überwachung von Narkosen während medizinischer Eingriffe, einzusetzen. Dabei wird ein Narkosemittel, beispielsweise Propofol, in der Atemluft eines Patienten kontinuierlich analysiert.

Außerdem sind aus dem Stand der Technik Analysevorrichtungen bekannt, welche ein lonenbeweglichkeitsspektrometer in Kombination mit einer vorgeschalteten Multikapillarsäule aufweisen. Eine Multikapillarsäule ist eine gaschromatographische Säule, welche aus einer Vielzahl von gebündelten Einzelkapillaren besteht, die unterschiedliche Analyten verschieden lange zurückhalten. In anderen Worten benötigen die Gasbestandteile der Exspirationsluft für einen Durchtritt durch die Multikapillarsäule unterschiedlich lange, so dass eine Exspirationsluft-Probe mittels der Multikapillarsäule vorgetrennt werden kann (1. Trennung). Eine Durchtrittsdauer durch die Multikapillarsäule wird dabei als Retentionszeit bezeichnet.

Nach der Vortrennung in der Multikapillarsäule gelangen die Gasbestandteile in das lonenbeweglichkeitsspektrometer, und zwar zunächst in einen lonisationskammerabschnitt des lonenbeweglichkeitsspektrometers, in welchem die Gasbestandteile der Exspirationsluft ionisiert werden. Dies geschieht mit Hilfe einer lonenquelle, beispielsweise mittels radioaktivem Nickel. Nach der Ionisierung durchlaufen die Ionen ein Sperrgitter und werden in einem Driftkammerabschnitt des lonenbeweglichkeitsspektrometers gegen die Strömungsrichtung eines Driftgases hin zu einer Detektionsvorrichtung beschleunigt. Ionen unterschiedlicher Masse bzw. Struktur erreichen hier unterschiedliche Driftgeschwindigkeiten, werden dadurch voneinander getrennt (2. Trennung) und treffen zeitlich nacheinander auf die Detektionsvorrichtung. Eine Durchtrittsdauer durch den Driftkammerabschnitt wird als Driftzeit bezeichnet. Die Beschleunigung der Ionen in dem lonenbeweglichkeitsspektrometer erfolgt mittels eines elektrischen Feldes. Die Analysevorrichtung gibt Signalausschläge aus, welche durch die auf die Detektionsvorrichtung treffenden, ionisierten Gasbestandteile der Exspirationsluft erzeugt werden.

Im Stand der Technik gelang es bei den Versuchen, derartige Analysevorrichtungen zum Einsatz am Patienten während eines medizinischen Eingriffs bereitzustellen, bisher nicht, mit der hohen relativen Feuchte von Exspirationsluft geeignet umzugehen. Bisher wurde die hohe Feuchte von Exspirationsluft immer als für die Messergebnisse schädlich betrachtet. Der Stand der Technik gibt lediglich Lösungen, wie die feuchtebedingten Einflüsse verringert/ umgangen werden können und trotz der hohen Feuchte von Exspirationsluft brauchbare Messergebnisse erzielt werden können. Weiterhin hat der Stand der Technik den Nachteil, dass die Analysevorrichtung nach einem Einschalten derselben erst nach einem Zeitraum von mehreren Tagen für quantitative Messungen zur Analyse von Exspirationsluft verwendet werden kann, da über diesen Zeitraum die ausgegebenen Signalausschläge kontinuierlich ansteigen und sich erst danach auf einen konstanten Wert einpendeln.

### Kurzbeschreibung der Erfindung

Vor diesem Hintergrund ist es Aufgabe der vorliegenden Erfindung, eine kompakt aufgebaute, anwendungsfalloptimierte Analysevorrichtung zum Analysieren von Exspirationsluft eines Patienten, vorzugsweise zum Überwachen einer Narkose des Patienten während eines medizinischen Eingriffs, bereitzustellen, mittels welcher verlässliche, exakte, wiederholgenaue und über einen längeren Zeitraum/ kontinuierliche Messungen unmittelbar nach einem Einschalten der Analysevorrichtung durchgeführt werden können.

Diese Aufgabe wird durch eine Analysevorrichtung nach Anspruch 1 gelöst. Vorteilhafte Weiterbildungen und Ausführungsformen sind Gegenstand der Unteransprüche und/oder nachfolgend erläutert.

Die Erfindung betrifft eine Analysevorrichtung zum Analysieren von Exspirationsluft eines Patienten, vorzugsweise zum Überwachen einer Narkose des Patienten während eines medizinischen Eingriffs, wobei die Analysevorrichtung konfiguriert ist, einen Anteil eines in der Exspirationsluft des Patienten enthaltenen Analyten in der Exspirationsluft zu bestimmen, mit: vorzugsweise einer Multikapillarsäule zum Vortrennen der zu analysierenden Exspirationsluft; und einem Ionenbeweglichkeitsspektrometer, in welchem Gasbestandteile der Exspirationsluft ionisiert und hin zu einer Detektionsvorrichtung beschleunigt werden; wobei die Analysevorrichtung Signalausschläge ausgibt, welche durch die auf die Detektionsvorrichtung treffenden, ionisierten Gasbestandteile der Exspirationsluft erzeugt werden. Ein Anteil des in der zu analysierenden Exspirationsluft enthaltenen, zu bestimmenden Analyten wird durch eine Kalibrierung des Signalausschlags des Analyten an einem Signalausschlag, welcher durch die Luftfeuchtigkeit von Exspirationsluft hervorgerufen wird, bestimmt.

Dabei ist es vorteilhaft, wenn ein maximaler Signalausschlag des Analyten in Verhältnis mit einem maximalen Signalausschlag des durch die Luftfeuchtigkeit von Exspirationsluft hervorgerufenen Signalausschlags gesetzt wird, und der Anteil des Analyten in der Exspirationsluft unter Annahme einer bekannten und konstanten relativen Luftfeuchtigkeit von Exspirationsluft des Patienten, insbesondere einer relativen Luftfeuchtigkeit von 95 % oder einer patientenspezifisch im Voraus ermittelten relativen Luftfeuchtigkeit, bestimmt wird.

In vorteilhafter Weise ist die Analysevorrichtung konfiguriert, durch die Kalibrierung des Signalausschlags des Analyten an dem durch die Luftfeuchtigkeit der Exspirationsluft hervorgerufenen Signalausschlag eine Anteilsmessung des Analyten in der Exspirationsluft bei kontinuierlich ansteigenden absoluten Signalausschlägen und somit unmittelbar nach einem Einschalten der Analysevorrichtung durchzuführen.

In anderen Worten wird gemäß der vorliegenden Erfindung zunächst ein absoluter, quantitativer, maximaler Wert eines Signalausschlags, welcher durch die Luftfeuchtigkeit von Exspirationsluft erzeugt wird, ermittelt. Aufgrund der hohen Feuchte von Exspirationsluft kann dieser Wert relativ einfach ermittelt werden, da lediglich der größte (maximale) von der Analysevorrichtung ausgegebene Signalausschlag gesucht werden muss. Es wird schließlich angenommen, dass dieser absolute Wert einem bekannten und konstanten Feuchtigkeitsanteil von Exspirationsluft des Patienten entspricht. Dieser Feuchtigkeitsanteil kann beispielsweise immer auf einen festen Wert, beispielsweise 95%, gesetzt werden oder vor dem jeweiligen medizinischen Eingriff patientenspezifisch ermittelt werden. Will man nun den Anteil eines spezifischen Analyten in der Exspirationsluft bestimmen, muss nur noch ein absoluter, quantitativer, maximaler Wert eines Signalausschlags des Analyten ermittelt werden. Über eine Dreisatz-Beziehung kann dann der Anteil des Analyten in der Exspirationsluft berechnet werden. Dieses beschriebene Zurückrechnen auf den Anteil des Analyten wird in der vorliegenden Erfindung als Kalibrierung bezeichnet.

Kern der vorliegenden Erfindung ist es somit, dass der bekannte und konstante Feuchteanteil von Exspirationsluft des Patienten zur Ermittlung des Anteils eines spezifischen Analyten genutzt wird. Weiterhin ist es Kern der vorliegenden Erfindung, dass für das Bestimmen des Anteils des Analyten lediglich das Verhältnis zwischen maximalem Signalausschlag des Analyten und maximalem feuchtebedingtem Signalausschlag bestimmt werden muss. Somit kann die erfindungsgemäße Analysevorrichtung auch bei kontinuierlich ansteigenden absoluten Werten, das heißt unmittelbar nach einem Einschalten der Analysevorrichtung, arbeiten.

Bevorzugt ist der zu bestimmende Analyt ein Anästhetikum, vorzugsweise Propofol. Wird ein Anteil eines Anästhetikums in der Exspirationsluft eines Patienten bestimmt, können Rückschlüsse auf eine Narkosetiefe gezogen und somit die Narkose des Patienten während eines medizinischen Eingriffs überwacht werden.

Es ist dabei von Vorteil, wenn die Analysevorrichtung konfiguriert ist, in vorbestimmten kurzen Zeitintervallen, insbesondere zumindest alle fünf Minuten, bevorzugt alle zwei Minuten, weiter bevorzugt jede Minute, den Anteil des Anästhetikums in der Exspirationsluft zu ermitteln und die erhaltenen Messwerte auf einem Display anzuzeigen.

Ein vorteilhaftes Ausführungsbeispiel ist dadurch gekennzeichnet, dass die Gasbestandteile der Exspirationsluft für einen Durchtritt durch die Multikapillarsäule unterschiedlich lange benötigen und eine Durchtrittsdauer durch die Multikapillarsäule als Retentionszeit bezeichnet wird; das lonenbeweglichkeitsspektrometer einen lonisationskammerabschnitt, in welchem die Gasbestandteile der Exspirationsluft ionisiert werden, und einen Driftkammerabschnitt, in welchem die ionisierten Gasbestandteile hin zu der Detektionsvorrichtung beschleunigt werden, aufweist und eine Durchtrittsdauer durch den Driftkammerabschnitt als Driftzeit bezeichnet wird; und die Analysevorrichtung die Signalausschläge in einem Chromatogramm in Abhängigkeit von der Retentionszeit und der Driftzeit ausgibt.

Bevorzugt liegt der durch die Luftfeuchtigkeit von Exspirationsluft hervorgerufene Signalausschlag im Wesentlichen unabhängig von der Retentionszeit nach einer bestimmten Driftzeit im Chromatogramm vor und stellt insbesondere einen maximalen Signalausschlag in dem Chromatogramm dar.

In vorteilhafter Weise wird der durch die Luftfeuchtigkeit der Exspirationsluft hervorgerufene Signalausschlag durch bei der Ionisierung der Exspirationsluft entstehende, auf die Detektionsvorrichtung treffende Reaktionsionen, insbesondere H⁺(H₂O)ⁿ-Ionen oder O₂⁻(H₂O)ⁿ-Ionen erzeugt, welche sich in dem Chromatogramm durch einen charakteristischen Signalausschlag auszeichnen.

Bevorzugt weist die Analysevorrichtung eine Datenbank auf, in welcher für verschiedene Analyten je zwei Werte für die Driftzeit und die Retentionszeit hinterlegt sind, wobei durch die vier in der Datenbank hinterlegten Werte ein Bereich in dem Chromatogramm definiert wird, in welchem sich der Signalausschlag für einen bestimmten Analyten befindet, wobei bevorzugt eine Driftzeitachse an dem durch die Luftfeuchtigkeit von Exspirationsluft hervorgerufenen Signalausschlag normiert ist.

### Kurzbeschreibung der Figuren

Die Erfindung wird nachfolgend mit Hilfe von Figuren weiter erläutert. Es zeigen:
- Fig. 1: eine schematische Darstellung einer erfindungsgemäßen Analysevorrichtung;
- Fig. 2: eine dreidimensionale Ansicht eines Chromatogramm, in welchem Signalausschläge in Abhängigkeit von einer Driftzeit und einer Retentionszeit dargestellt sind;
- Fig. 3: eine zweidimensionale Ansicht eines Chromatogramms, in welchem Signalausschläge in Abhängigkeit von der Driftzeit und der Retentionszeit dargestellt sind;
- Fig. 4: eine Displayanzeige der erfindungsgemäßen Analysevorrichtung; und
- Fig. 5: ein Flussdiagramm von durchzuführenden Schritten bis die erfindungsgemäße Analysevorrichtung messbereit ist.

### Figurenbeschreibung

Die Figuren sind lediglich schematischer Natur und dienen ausschließlich dem Verständnis der Erfindung. Gleiche Elemente sind dabei mit denselben Bezugszeichen versehen.

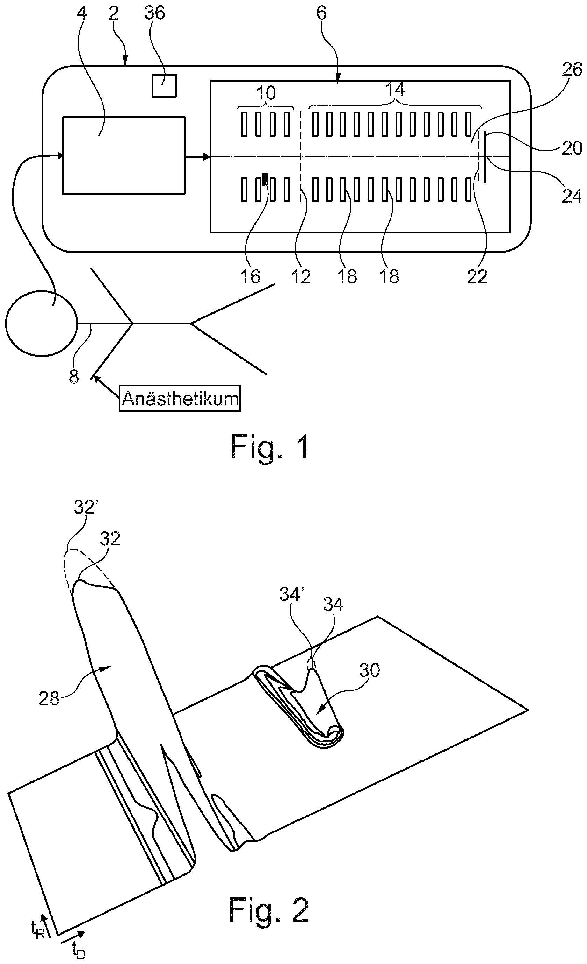

Fig. 1 zeigt eine schematische Darstellung einer erfindungsgemäßen Analysevorrichtung 2. Die Analysevorrichtung 2 weist eine Multikapillarsäule 4 und ein lonenbeweglichkeitsspektrometer 6 auf. Die Multikapillarsäule 4 besteht aus einer Vielzahl von gebündelten Einzelkapillaren (nicht dargestellt). Verschiedene Gasbestandteile von Exspirationsluft benötigen für einen Durchtritt durch die Multikapillarsäule 4 unterschiedlich lange. Exspirationsluft, welche von einem Patienten 8 ausgestoßen wird und wie in Fig. 1 gezeigt der Multikapillarsäule 4 zugeführt wird, wird somit mit Hilfe der Multikapillarsäule in einzelne Gasbestandteile aufgetrennt. Die Zeit, welche ein Gasbestandteil für die Passage durch die Multikapillarsäule 4 benötigt, wird als Retentionszeit t_{R} bezeichnet.

Nach einer ersten Trennung mittels der Multikapillarsäule 4 wird die Exspirationsluft bzw. werden die Gasbestandteile derselben dem lonenbeweglichkeitsspektrometer 6 zugeführt. Das lonenbeweglichkeitsspektrometer 6 umfasst einen lonisationskammerabschnitt 10 und einen daran angrenzenden, durch ein Bradbury-Nielsen-Gitter 12 von dem lonisationskammerabschnitt 10 getrennten Driftkammerabschnitt 14. In dem lonisationskammerabschnitt 10 werden die Gasbestandteile der Exspirationsluft mittels einer lonisationsquelle 16 (beispielsweise radioaktives Nickel) ionisiert. Das Bradbury-Nielsen-Gitter 12 steuert ein Eindringen der in dem lonisationskammerabschnitt 10 erzeugten Ionen in den Driftkammerabschnitt 14. Über ein mittels Hochspannungsringe 18 erzeugtes elektrisches Feld werden die Ionen hin zu einer Faraday-Platte 20 beschleunigt, welche einer Detektion der Ionen dient. Unmittelbar vor der Faraday-Platte 20 ist ein Apertur-Gitter 22 als Abschirmgitter zur kapazitiven Entkopplung der Ionen vorgesehen. An der die Faraday-Platte 20 aufweisenden Seite des Driftkammerabschnitts 14 ist eine Einlassöffnung 24 für ein Driftgas vorgesehen, welches einen Innenraum 26 entgegen einer Driftrichtung der Ionen durchströmt und verhindert, dass ungeladene Moleküle oder Partikel in den Driftkammerabschnitt 14 hinein gelangen.

Ionen unterschiedlicher Masse bzw. Struktur erreichen in dem Driftkammerabschnitt 14 unterschiedliche Driftgeschwindigkeiten, werden dadurch voneinander getrennt (zweite Trennung) und treffen zeitlich nacheinander auf die Faraday-Platte 20. Eine Durchtrittsdauer der Ionen durch den Driftkammerabschnitt 14 wird dabei als Driftzeit t_{D} bezeichnet.

Die Analysevorrichtung 2 ist konfiguriert, einen Anteil eines in der Exspirationsluft enthaltenen Analyten in der Exspirationsluft zu bestimmen. Der zu bestimmende Analyt ist in der vorliegenden Erfindung bevorzugt ein Anästhetikum, vorzugsweise Propofol, welches dem Patienten 8 intravenös verabreicht wurde und welches dieser im narkotisierten Zustand über die Exspirationsluft ausatmet.

Wie genau der Anteil des in der Exspirationsluft enthaltenen Analyten erfindungsgemäß bestimmt wird, wird mit Bezugnahme auf Fig. 2 erläutert. In Fig. 2 ist ein Chromatogramm dargestellt, welches beispielhaft zwei Signalausschläge aufweist, welche durch auf die Faraday-Platte 20 treffende, ionisierte Gasbestandteile erzeugt werden und von der Analysevorrichtung 2 in Abhängigkeit von der Retentionszeit t_{R} und der Driftzeit t_{D} ausgegeben werden.

In Fig. 2 ist ein erster Signalausschlag 28 und ein zweiter Signalausschlag 30 dargestellt. Der erste Signalausschlag 28 wird durch die Luftfeuchtigkeit der Exspirationsluft hervorgerufen, insbesondere durch bei der Ionisierung der Exspirationsluft entstehende, auf die Faraday-Platte 20 treffende Reaktionsionen, insbesondere H⁺(H₂O)ⁿ-Ionen oder O₂⁻(H₂O)ⁿ-Ionen. Dieser erste Signalausschlag 28 liegt im Wesentlichen unabhängig von der Retentionszeit nach einer bestimmten Driftzeit im Chromatogramm vor und stellt aufgrund der hohen relativen Feuchte von Exspirationsluft von über 95% bei einer Analyse von Exspirationsluft immer einen maximalen, charakteristischen Signalausschlag in dem Chromatogramm dar.

Der zweite Signalausschlag 30 wird durch einen Analyten (beispielsweise ein Anästhetikum, vorzugsweise Propofol) hervorgerufen, dessen Anteil in der Exspirationsluft zu bestimmen ist.

Erfindungsgemäß wird zunächst ein erstes Maximum (absoluter, quantitativer Wert) 32 des ersten Signalausschlags 28 bestimmt. Anschließend wird ein zweites Maximum (absoluter, quantitativer Wert) 34 des zweiten Signalausschlags 30 bestimmt. Um nun den Anteil des Analyten in der Exspirationsluft zu erhalten, wird beispielsweise das Verhältnis zwischen zweitem Maximum 34 und erstem Maximum 32 gebildet und mit dem bekannten und konstanten Luftfeuchtigkeitsanteil der Exspirationsluft des Patienten 8 multipliziert. In anderen Worten erfolgt erfindungsgemäß eine Kalibrierung des zweiten Maximums 34 des zweiten Signalausschlags 30 an dem ersten Maximum 32 des ersten Signalausschlags 30.

In Fig. 2 sind in gestrichelten Linien noch ein erstes Maximum 32' des ersten Signalausschlags 28 und ein zweites Maximum 34' des zweiten Signalausschlags 30 angedeutet. Das erste Maximum 32' und das zweite Maximum 34' würde erhalten werden, wenn dieselbe Exspirationsluft-Probe der Analysevorrichtung 2 zu einem späteren Zeitpunkt noch einmal zugeführt werden würde. In anderen Worten werden grundsätzlich mit zunehmender Zeitdauer nach einem Einschalten der Analysevorrichtung 2 zunehmende Maxima 32', 34' der Signalausschläge 28, 30 erhalten. Die vorliegende Erfindung ermöglicht es, geeignet mit kontinuierlich ansteigenden absoluten Signalausschlägen umzugehen, da erfindungsgemäß immer nur das Verhältnis zwischen zweitem Maximum 34, 34' und erstem Maximum 32, 32' zu bilden ist.

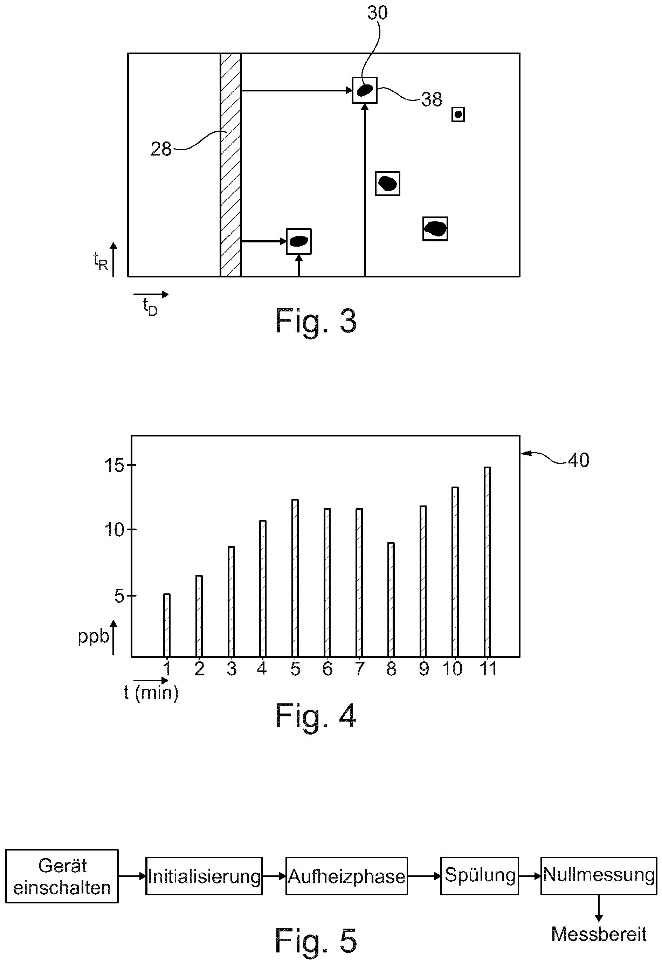

In Fig. 3 ist eine zweidimensionale Ansicht des erhaltenen Chromatogramms dargestellt, in welchem verschiedene erhaltene Signalausschläge, beispielsweise wiederum die in Fig. 2 dargestellten Signalausschläge 28 und 30 dargestellt sind. Wie voranstehend schon erläutert, stellt der Signalausschlag 28 einen für die Feuchte charakteristischen Signalausschlag dar und kann in einfacher Weise gefunden werden, da er nach einer bestimmten Driftzeit t_{D} unabhängig von der Retentionszeit t_{R} vorliegt.

Die Analysevorrichtung 2 enthält eine Datenbank 36, in welcher für verschiedene zu bestimmende Analyten je zwei Werte für die Driftzeit t_{D} und die Retentionszeit t_{R} hinterlegt sind. Durch diese vier Werte wird in dem Chromatogramm ein rechteckiger Bereich 38 definiert, in welchem sich der Signalausschlag für einen bestimmten Analyt befindet. Bevorzugt sind die Werte für die Driftzeit t_{D} dabei an dem ersten, charakteristischen Signalausschlag 28 normiert.

Soll nun das zweite Maximum 34 des zweiten Signalausschlags 30 bestimmt werden, wird durch die in der Datenbank 36 enthaltenen Werte der rechteckige Bereich 38 in dem Chromatogramm definiert und es muss nur noch das Maximum/ der maximale absolute Wert in dem definierten rechteckigen Bereich 38 bestimmt werden.

Wie in Fig. 4 gezeigt misst/berechnet/ermittelt die Analysevorrichtung 2 beispielsweise jede Minute einen Anteil ppb (parts per billion) eines Analyten, vorzugsweise eines Anästhetikums, in der Exspirationsluft eines Patienten und gibt den jeweils erhaltenen Anteil auf einem Display aus. Dadurch wird es einem Arzt ermöglicht, kontinuierlich den Anteil des Anästhetikums (Propofol) in der Exspirationsluft zu analysieren und eine Narkose des Patienten während eines medizinischen Eingriffs zu überwachen. Beispielsweise kann der Arzt, wenn er wie in Fig. 4 dargestellt nach 6 min bzw. 7 min merkt, dass der Anteil des Anästhetikums in der Exspirationsluft zurückgeht, dem Patienten 8 das Anästhetikum erneut intravenös verabreichen.

Fig. 5 zeigt ein Flussdiagramm von durchzuführenden Schritten bis die erfindungsgemäße Analysevorrichtung 2 messbereit ist. Nach einem Einschalten des Geräts folgen eine Initialisierung, eine Aufheizphase, eine Spülung und eine Nullmessung. Diese Schritte dauern weniger als 30 min. Wenn voranstehend davon die Rede war, dass eine Anteilsmessung des Analyten in der Exspirationsluft unmittelbar nach einem Einschalten der Analysevorrichtung 2 durchgeführt werden kann, ist somit gemeint, dass eine Anteilsmessung spätestens nach 30 min durchgeführt werden kann.

### Bezugszeichenliste

- 2: Analysevorrichtung
- 4: Multikapillarsäule
- 6: Ionenbeweglichkeitsspektrometer
- 8: Patient
- 10: Ionisationskammerabschnitt
- 12: Bradbury-Nielsen-Gitter
- 14: Driftkammerabschnitt
- 16: lonisationsquelle
- 18: Hochspannungsring
- 20: Faraday-Platte
- 22: Apertur-Gitter
- 24: Einlassöffnung
- 26: Innenraum
- 28: erster Signalausschlag
- 30: zweiter Signalausschlag
- 32, 32': erstes Maximum
- 34, 34': zweites Maximum
- 36: Datenbank
- 38: rechteckiger Bereich
- 40: Display

## Patentansprüche

1. Analysevorrichtung (2), angepasst zum Analysieren von Exspirationsluft eines Patienten (8), vorzugsweise zum Überwachen einer Narkose des Patienten (8) während eines medizinischen Eingriffs, wobei die Analysevorrichtung (2) konfiguriert ist, einen Anteil eines in der Exspirationsluft des Patienten (8) enthaltenen Analyten in der Exspirationsluft zu bestimmen, mit: zumindest einer Multikapillarsäule (4), angepasst zum Vortrennen der zu analysierenden Exspirationsluft; und einem lonenbeweglichkeitsspektrometer (6), in welchem Gasbestandteile der Exspirationsluft ionisiert und hin zu einer Detektionsvorrichtung (20) beschleunigt werden; wobei die Analysevorrichtung (2) angepasst ist, Signalausschläge (28, 30) auszugeben, welche durch die auf die Detektionsvorrichtung (20) treffenden, ionisierten Gasbestandteile der Exspirationsluft erzeugt werden,
**gekennzeichnet durch**
eine Bestimmungseinheit (CPU), die dafür angepasst ist, einen Anteil des in der zu analysierenden Exspirationsluft enthaltenen, zu bestimmenden Analyten durch eine Kalibrierung des Signalausschlags (30) des Analyten an einem Signalausschlag (28), welcher durch die Luftfeuchtigkeit von Exspirationsluft hervorgerufen wird, zu bestimmen.

2. Analysevorrichtung (2) nach Anspruch 1, **dadurch gekennzeichnet, dass** ein maximaler Signalausschlag (34, 34') des Analyten in Verhältnis mit einem maximalen Signalausschlag (32, 32') des durch die Luftfeuchtigkeit von Exspirationsluft hervorgerufenen Signalausschlags (28) gesetzt wird, und der Anteil des Analyten in der Exspirationsluft unter Annahme einer bekannten und konstanten relativen Luftfeuchtigkeit von Exspirationsluft, insbesondere einer relativen Luftfeuchtigkeit von 95 % oder einer patientenspezifisch im Voraus ermittelten relativen Luftfeuchtigkeit, bestimmt wird.

3. Analysevorrichtung (2) nach Anspruch 1 oder 2, **dadurch gekennzeichnet, dass** diese konfiguriert ist, durch die Kalibrierung des Signalausschlags (30) des Analyten an dem durch die Luftfeuchtigkeit der Exspirationsluft hervorgerufenen Signalausschlag (28) eine Anteilsmessung des Analyten in der Exspirationsluft bei kontinuierlich ansteigenden absoluten Signalausschlägen (32, 32', 34, 34') und somit unmittelbar nach einem Einschalten der Analysevorrichtung (2) durchzuführen.

4. Analysevorrichtung (2) nach einem der vorhergehenden Ansprüche, **dadurch gekennzeichnet, dass** der zu bestimmende Analyt ein Anästhetikum, vorzugsweise Propofol, ist.

5. Analysevorrichtung (2) nach Anspruch 4, **dadurch gekennzeichnet, dass** diese konfiguriert ist, in vorbestimmten kurzen Zeitintervallen, insbesondere zumindest alle fünf Minuten, bevorzugt alle zwei Minuten, weiter bevorzugt jede Minute, den Anteil des Anästhetikums in der Exspirationsluft zu ermitteln und die erhaltenen Messwerte auf einem Display (40) anzuzeigen.

6. Analysevorrichtung (2) nach einem der vorhergehenden Ansprüche, wobei
die Gasbestandteile der Exspirationsluft für einen Durchtritt durch eine Multikapillarsäule (4) unterschiedlich lange benötigen und eine Durchtrittsdauer durch die Multikapillarsäule (4) als Retentionszeit (t_{R}) bezeichnet wird; wobei
das lonenbeweglichkeitsspektrometer (6) einen lonisationskammerabschnitt (10), in welchem die Gasbestandteile der Exspirationsluft ionisiert werden, und einen Driftkammerabschnitt (14), in welchem die ionisierten Gasbestandteile hin zu der Detektionsvorrichtung (20) beschleunigt werden, aufweist und eine Durchtrittsdauer durch den Driftkammerabschnitt (14) als Driftzeit (t_{D}) bezeichnet wird; und wobei
die Analysevorrichtung (2) die Signalausschläge (28, 30) in einem Chromatogramm in Abhängigkeit von der Retentionszeit (t_{R}) und der Driftzeit (t_{D}) ausgibt.

7. Analysevorrichtung (2) nach Anspruch 6, **dadurch gekennzeichnet, dass** der durch die Luftfeuchtigkeit von Exspirationsluft hervorgerufene Signalausschlag (28) im Wesentlichen unabhängig von der Retentionszeit (t_{R}) nach einer bestimmten Driftzeit (t_{D}) im Chromatogramm vorliegt und einen maximalen Signalausschlag (32, 32') in dem Chromatogramm darstellt.

8. Analysevorrichtung (2) nach Anspruch 6 oder 7, **dadurch gekennzeichnet, dass** der durch die Luftfeuchtigkeit der Exspirationsluft hervorgerufene Signalausschlag (28) durch bei der Ionisierung der Exspirationsluft entstehende, auf die Detektionsvorrichtung (20) treffende Reaktionsionen, insbesondere H⁺(H₂O)ⁿ-Ionen oder O₂-(H₂O)ⁿ-Ionen erzeugt wird, welche sich in dem Chromatogramm durch einen charakteristischen Signalausschlag (28) auszeichnen.

9. Analysevorrichtung (2) nach einem der Ansprüche 6 bis 8, ferner **gekennzeichnet durch** eine Datenbank (36), in welcher für verschiedene Analyten je zwei Werte für die Driftzeit (t_{D}) und die Retentionszeit (t_{R}) hinterlegt sind, wobei durch diese vier in der Datenbank (36) hinterlegten Werte ein Bereich (38) in dem Chromatogramm definiert wird, in welchem sich der Signalausschlag (30) für einen bestimmten Analyten befindet, wobei bevorzugt eine Driftzeitachse an dem durch die Luftfeuchtigkeit von Exspirationsluft hervorgerufenen Signalausschlag (28) normiert ist.

## Claims

1. Analysis device (2) adapted for analyzing expiration air of a patient (8), preferably for monitoring an anesthesia of the patient (8) during a medical intervention, the analysis device (2) being configured to determine, in the expiration air, a portion of an analyte contained in the expiration air of the patient (8), comprising: at least one multi-capillary column (4) adapted for pre-separating the expiration air to be analyzed; and an ion mobility spectrometer (6) in which gas components of the expiration air are ionized and accelerated towards a detection device (20); the analysis device (2) being adapted to output signal excursions (28, 30) which are created by the ionized gas components of the expiration air which hit the detection device (20),
**characterized by**
a determination device (CPU) that is adapted to determine a portion of the analyte which is to be determined and is contained in the expiration air to be analyzed by calibration of the signal excursion (30) of the analyte to a signal excursion (28) which is caused by the air moisture of expiration air.

2. Analysis device (2) according to claim 1, **characterized in that** a maximum signal excursion (34, 34') of the analyte is put in relation to a maximum signal excursion (32, 32') of the signal excursion (28) caused by the air moisture of expiration air, and **in that** the portion of the analyte in the expiration air is determined on the assumption of a known and constant relative air moisture of expiration air, especially a relative air moisture of 95% or a relative air moisture established in advance for a specific patient.

3. Analysis device (2) according to claim 1 or 2, **characterized in that** the analysis device is configured to carry out measurement of a portion of the analyte in the expiration air by calibration of the signal excursion (30) of the analyte to the signal excursion (28) caused by the air moisture of the expiration air with continuously increasing absolute signal excursions (32, 32', 34, 34') and thus immediately after turning on the analysis device (2).

4. Analysis device (2) according to any one of the preceding claims, **characterized in that** the analyte to be determined is an anesthetic, preferably propofol.

5. Analysis device (2) according to claim 4, **characterized in that** the analysis device is configured to establish the portion of the anesthetic in the expiration air at predetermined short time intervals, especially at least every five minutes, preferably every two minutes, further preferred every minute, and to indicate the measuring values obtained on a display (40).

6. Analysis device (2) according to any one of the preceding claims, wherein
the gas components of the expiration air take differently long times for a passage through a multi-capillary column (4) and a passage time through the multi-capillary column (4) is referred to as retention time (t_{R}); wherein
the ion mobility spectrometer (6) has an ionization chamber section (10) in which the gas components of the expiration air are ionized and a drift chamber section (14) in which the ionized gas components are accelerated toward the detection device (20), and a passage time through the drift chamber section (14) is referred to as drift time (t_{D}); and wherein
the analysis device (2) outputs the signal excursions (28, 30) in a chromatogram as a function of the retention time (t_{R}) and the drift time (t_{D}).

7. Analysis device (2) according to claim 6, **characterized in that** the signal excursion (28) caused by the air moisture of expiration air is provided in the chromatogram substantially independently of the retention time (t_{R}) after a particular drift time (t_{D}) and represents a maximum signal excursion (32, 32') in the chromatogram.

8. Analysis device (2) according to claim 6 or 7, **characterized in that** the signal excursion (28) caused by the air moisture of the expiration air is created by reaction ions, especially H⁺(H₂O)ⁿ ions or O₂⁻(H₂O)ⁿ ions, formed during ionization of the expiration air and hitting the detection device (20) which excel by a characteristic signal excursion (28) in the chromatogram.

9. Analysis device (2) according to any one of the claims 6 to 8, further **characterized by** a data base (36) in which two values each for the drift time (t_{D}) and the retention time (t_{R}) are stored for different analytes, wherein said four values stored in the data base (36) define a range (38) in the chromatogram in which the signal excursion (30) for a particular analyte is provided, wherein preferably a drift time axis is scaled to the signal excursion (28) caused by the air moisture of expiration air.

## Revendications

1. Dispositif d'analyse (2), adapté pour l'analyse d'air expiré d'un patient (8), de préférence pour la surveillance d'une anesthésie du patient (8) pendant une intervention médicale, dans lequel le dispositif d'analyse (2) est configuré afin de déterminer une part d'un analyte contenu dans l'air expiré du patient (8) dans l'air expiré, avec : au moins une colonne à capillaires multiples (4), adaptée pour la séparation préalable de l'air expiré à analyser ; et un spectromètre à mobilité ionique (6) dans lequel des constituants gazeux de l'air expiré sont ionisés et sont accélérés vers un dispositif de détection (20) ; dans lequel le dispositif d'analyse (2) est adapté afin d'émettre des fluctuations de signal (28, 30) qui sont générées par les constituants gazeux ionisés, rencontrant le dispositif de détection (20) de l'air expiré,
**caractérisé par**
une unité de détermination (CPU) qui est adaptée afin de déterminer une part de l'analyte à déterminer, contenu dans l'air expiré à analyser par un calibrage de la fluctuation de signal (30) de l'analyte au niveau d'une fluctuation de signal (28) qui est suscitée par l'humidité de l'air de l'air expiré.

2. Dispositif d'analyse (2) selon la revendication 1, **caractérisé en ce qu'**une fluctuation de signal (34, 34') maximale de l'analyte est placée en rapport avec une fluctuation de signal (32, 32') maximale de la fluctuation de signal (28) suscitée par l'humidité de l'air de l'air expiré, et la part de l'analyte dans l'air expiré est déterminée dans l'hypothèse d'une humidité de l'air relative connue et constante de l'air expiré, en particulier d'une humidité de l'air relative de 95 % ou d'une humidité de l'air relative calculée au préalable de manière spécifique au patient.

3. Dispositif d'analyse (2) selon la revendication 1 ou 2, **caractérisé en ce que** celui-ci est configuré afin de réaliser par le calibrage de la fluctuation de signal (30) de l'analyte au niveau de la fluctuation de signal (28) suscitée par l'humidité de l'air de l'air expiré une mesure de part de l'analyte dans l'air expiré pour des fluctuations de signal (32, 32', 34, 34') absolues croissantes en continu et ainsi directement après une mise en marche du dispositif d'analyse (2).

4. Dispositif d'analyse (2) selon l'une quelconque des revendications précédentes, **caractérisé en ce que** l'analyte à déterminer est un anesthésique, de préférence du Propofol.

5. Dispositif d'analyse (2) selon la revendication 4, **caractérisé en ce que** celui-ci est configuré afin de calculer, dans des intervalles de temps courts prédéterminés, en particulier au moins toutes les cinq minutes, de préférence toutes les deux minutes, de manière encore préférée chaque minute, la part de l'anesthésique dans l'air expiré et d'afficher les valeurs de mesure obtenues sur un écran (40).

6. Dispositif d'analyse (2) selon l'une quelconque des revendications précédentes, dans lequel
les constituants gazeux de l'air expiré pour un passage par une colonne à capillaires multiples (4) nécessitent une longueur différente et une durée de passage par la colonne à capillaires multiples (4) est désignée par temps de rétention (t_{R}) ; dans lequel
le spectromètre à mobilité ionique (6) présente une section de chambre d'ionisation (10), dans laquelle les constituants gazeux de l'air expiré sont ionisés, et une section de chambre de dérive (14), dans laquelle les constituants gazeux ionisés sont accélérés jusqu'au dispositif de détection (20), et une durée de passage par la section de chambre de dérive (14) est désignée par temps de dérive (t_{D}) ; et dans lequel
le dispositif d'analyse (2) émet les fluctuations de signal (28, 30) dans un chromatogramme en fonction du temps de rétention (t_{R}) et du temps de dérive (t_{D}).

7. Dispositif d'analyse (2) selon la revendication 6, **caractérisé en ce que** la fluctuation de signal (28) suscitée par l'humidité de l'air de l'air expiré se présente sensiblement indépendamment du temps de rétention (t_{R}) après un temps de dérive déterminé (t_{D}) dans le chromatogramme et représente une fluctuation de signal (32, 32') maximale dans le chromatogramme.

8. Dispositif d'analyse (2) selon la revendication 6 ou 7, **caractérisé en ce que** la fluctuation de signal (28) suscitée par l'humidité de l'air de l'air expiré est générée par des ions réactifs rencontrant le dispositif de détection (20), apparaissant lors de l'ionisation de l'air expiré, en particulier des ions H⁺(H₂O)ⁿ ou des ions O₂⁻(H₂O)ⁿ qui se distinguent dans le chromatogramme par une fluctuation de signal (28) caractéristique.

9. Dispositif d'analyse (2) selon l'une quelconque des revendications 6 à 8, **caractérisé de plus par** une base de données (36) dans laquelle pour différents anesthésiques deux valeurs pour le temps de dérive (t_{D}) et le temps de rétention (t_{R}) sont chaque fois enregistrées, dans lequel par ces quatre valeurs enregistrées dans la base de données (36) une zone (38) est définie dans le chromatogramme, dans laquelle se trouve la fluctuation de signal (30) pour un analyte déterminé, dans lequel de préférence un axe de temps de dérive est normé au niveau de la fluctuation de signal (28) suscitée par l'humidité de l'air de l'air expiré.
